# FASCICULE DE BREVET EUROPEEN

(11) **EP 2 825 629 B1**
(45) Date de publication et mention de la délivrance du brevet: **18.10.2017**
(21) Numéro de dépôt: 13715325.0
(22) Date de dépôt: 15.03.2013
(51) Int. Cl.: C12N 1/12, C12N 13/00, C12P 7/64, C12P 23/00

(54) **PRODUCTION D'ACIDE DOCOSAHEXAENOIQUE ET/OU D'ACIDE EICOSAPENTAENOIQUE ET/OU DE CAROTENOIDES EN MODE MIXOTROPHE PAR NITZSCHIA**
HERSTELLUNG VON DOCOSAHEXAENSÄURE UND/ODER EICOSAPENTAENSÄURE UND/ODER CAROTINOIDEN IN EINEM MIXOTROPHEN MODUS MITHILFE VON NITZSCHIA
PRODUCTION OF DOCOSAHEXAENOIC ACID AND/OR EICOSAPENTAENOIC ACID AND/OR CAROTENOIDS IN MIXOTROPHIC MODE USING NITZSCHIA

(30) Priorité: 16.03.2012 FR 1252378; 16.08.2012 FR 1257843
(43) Date de publication de la demande: 21.01.2015
(73) Titulaire: Fermentalg, 33500 Libourne (FR)
(72) Inventeur: ROMARI, Khadidja, 34200 Sète (FR); GODART, François, 33870 Vayres (FR); CALLEJA, Pierre, 33500 Libourne (FR)
(74) Mandataire: Be IP
(86) Numéro de dépôt international: PCT/FR2013/050542
(87) Numéro de publication internationale: WO 2013/136025

(56) Documents cités:
- WO-A1-96/21723
- WO-A1-2009/134114
- CHU WAN-LOY ET AL: "Environmental effects on growth and biochemical composition of Nitzschia inconspicua Grunow", JOURNAL OF APPLIED PHYCOLOGY, vol. 8, no. 4-5, 1996, pages 389-396, XP055047175, & 7TH INTERNATIONAL CONFERENCE OF THE INTERNATIONAL ASSOCIATION OF APPLIED ALGOLOGY; KNYSNA, SOUTH AFRICA; APRIL 16-19, 1996 ISSN: 0921-8971
- ZHI-YOU WEN ET AL: "Production potential of eicosapentaenoic acid by the diatom Nitzschia laevis", BIOTECHNOLOGY LETTERS, SPRINGER NETHERLANDS, DORDRECHT, vol. 22, no. 9, 1 mai 2000 (2000-05-01), pages 727-733, XP019230749, ISSN: 1573-6776, DOI: 10.1023/A:1005666219163
- CERON GARCIA M C ET AL: "Mixotrophic production of marine microalga Phaeodactylum tricornutum on various carbon sources", JOURNAL OF MICROBIOLOGY AND BIOTECHNOLOGY, vol. 16, no. 5, mai 2006 (2006-05), pages 689-694, XP002688890,
- PENNINGTON F ET AL: "Carotenoid Distribution Patterns in Bacillariophyceae (Diatoms)", BIOCHEMICAL SYSTEMATICS AND ECOLOGY, PERGAMON PRESS, GB, vol. 16, no. 7-8, 14 décembre 1988 (1988-12-14), pages 589-592, XP023532490, ISSN: 0305-1978, DOI: 10.1016/0305-1978(88)90067-1 [extrait le 1988-12-14]
- WEN Z-Y ET AL: "CONTINUOUS CULTIVATION OF THE DIATOM NITZSCHIA LAEVIS FOR EICOSAPENTAENOIC ACID PRODUCTION: PHYSIOLOGICAL STUDY AND PROCESS OPTIMIZATION", BIOTECHNOLOGY PROGRESS, AMERICAN INSTITUTE OF CHEMICAL ENGINEERS, US, vol. 18, 27 December 2001 (2001-12-27), pages 21-28, XP009070409, ISSN: 8756-7938, DOI: 10.1021/BP010125N
- SANG MIN KIM ET AL: "Fucoxanthin as a major carotenoid in Isochrysis aff. galbana: Characterization of extraction for commercial application", JOURNAL OF THE KOREAN SOCIETY FOR APPLIED BIOLOGICAL CHEMISTRY, vol. 55, no. 4, 31 August 2012 (2012-08-31), pages 477-483, XP055049996, ISSN: 1738-2203, DOI: 10.1007/s13765-012-2108-3
- TORNABENE T G ET AL: "Sterols, aliphatic hydrocarbons, and fatty acids of a nonphotosynthetic diatom", LIPIDS, SPRINGER-VERLAG, BERLIN/HEIDELBERG, vol. 9, no. 4, 1 April 1974 (1974-04-01), pages 279-284, XP035174223, ISSN: 1558-9307, DOI: 10.1007/BF02532206

## Description

L'invention se rapporte à un procédé de culture en mode mixotrophe, notamment en présence d'un éclairement discontinu et/ou variable de lumière, d'une microalgue du genre *Nitzschia,* en particulier de l'espèce *Nitzschia brevirostris.* Le procédé permet d'obtenir un haut rendement en biomasse et un enrichissement des microalgues ainsi cultivées en lipides et plus particulièrement en acide docosahexaénoïque (DHA) et/ou en acide éicosapentaénoïque (EPA). Le procédé permet également d'obtenir un enrichissement des microalgues ainsi cultivées en caroténoïdes, et plus particulièrement, en fucoxanthine. Le procédé permet ainsi de sélectionner des souches de *Nitzschia,* en particulier de *Nitzschia brevirostris,* à caractère mixotrophe, et ayant un haut rendement en lipides et plus particulièrement en acides gras polyinsaturés, et/ou un haut rendement en caroténoïdes, plus particulièrement, en fucoxanthine. L'invention se rapporte aussi à une nouvelle souche de microalgue appartenant à l'espèce *Nitzschia brevirostris,* particulièrement adaptée à la production d'acides gras et de caroténoïdes. Cette nouvelle souche de *Nitzschia brevirostris* est utile pour produire de l'acide docosahexaénoïque (DHA) et/ou l'acide éicosapentaénoïque (EPA) et/ou de la fucoxanthine en mode mixotrophe.

### Préambule

Les microalgues sont des microorganismes photosynthétiques à caractère autotrophe, c'est-à-dire ayant l'aptitude de croître de manière autonome par photosynthèse.

Les microalgues se développent aussi bien dans les milieux aquatiques marins qu'en eaux douces ou saumâtres, ainsi que dans divers habitats terrestres.

La plupart des espèces de microalgues rencontrées dans l'eau douce ou les océans sont généralement autotrophes, c'est-à-dire qu'elles ne peuvent croître que par photosynthèse. Pour celles-ci, la présence dans leur milieu de substrats carbonés organiques ou de matière organique ne leur est pas favorable et n'améliore pas leur croissance. Cependant, un certain nombre d'espèces de microalgues, de familles et d'origines très diverses, s'avèrent ne pas être strictement autotrophes. C'est ainsi que certaines d'entre elles, dites hétérotrophes, sont capables de se développer en l'absence totale de lumière, par fermentation, c'est-à-dire en exploitant la matière organique.

D'autres espèces de microalgues, pour lesquelles la photosynthèse reste indispensable à leur développement, sont capables à la fois de tirer parti de la photosynthèse et de la matière organique présente dans leur milieu. Ces espèces intermédiaires, dites mixotrophes, peuvent être cultivées à la fois en présence de lumière et de matière organique.

Cette particularité des algues dites « mixotrophes » semble être liée à leur métabolisme, qui leur permet d'opérer simultanément photosynthèse et fermentation. Les deux types de métabolisme coexistent avec un effet global positif sur la croissance des algues [Yang, C. et al. (2000) ; Biochemical Engineering Journal, 6 :87-102].

Les microalgues font l'objet actuellement de nombreux projets industriels car certaines espèces sont capables d'accumuler ou de secréter des quantités importantes de lipides, notamment d'acides gras polyinsaturés.

Parmi ces acides gras polyinsaturés, certains hautement insaturés (AGHI) de la série des oméga-3 (PUFA-ω3), en particulier l'acide éicosapentaénoïque (EPA ou C20:5 ω3) et l'acide docosahexaénoïque (DHA ou C22:6 ω3), et de la série des oméga-6 (PUFA-ω6), en particulier, l'acide arachidonique (ARA ou AA ou encore acide eicosatétraènoïque C20:4 ω6) ont une importance nutritionnelle reconnue et présentent de fortes potentialités en terme d'applications thérapeutiques.

Considéré comme nutriment essentiel, le DHA est nécessaire au développement normal et fonctionnel des cellules, et joue un rôle crucial dans divers processus et fonctions biochimiques. Sa nature polyinsaturée lui confère une importance cruciale vis-à-vis des propriétés de la membrane cellulaire, chez les végétaux comme chez les animaux : fluidité, flexibilité et perméabilité sélective permettant par exemple une adaptation effective, et même la survie, aux basses températures, en particulier chez les poissons.

Le DHA est un constituant structurel majeur du cerveau humain. Le DHA représente 15-20 % du cortex cérébral (le cerveau d'un adulte contient au moins 20 g de DHA) et 30-60 % de la rétine. Il est indispensable au développement du système nerveux central et à la fonction rétinienne, par incorporation dans les membranes cellulaires, et joue un rôle capital dans l'acquisition et le maintien satisfaisant des mécanismes de la vision et de la mémoire.

Les huiles de poissons, issues de l'industrie de la pêche, sont actuellement la principale source commerciale de ce type d'acides gras. Toutefois, alors que ces huiles trouvent de nouvelles applications (complément alimentaire en aquaculture, incorporation dans les margarines), les ressources halieutiques marines se raréfient du fait d'une activité de pêche intensive.

De nouvelles sources de ces acides gras tels que l'EPA, le DHA et l'ARA doivent donc être recherchées afin de répondre, dans le futur, à la demande croissante du marché pour ce type d'acides gras polyinsaturés.

Outre leur capacité à synthétiser les acides gras *de novo,* les microalgues offrent plusieurs avantages par rapport aux huiles de poisson : elles sont cultivables *in vitro* dans des conditions contrôlées, ce qui permet la production d'une biomasse de composition biochimique relativement constante et, d'autre part, contrairement aux huiles de poissons, elles ne présentent pas d'odeur désagréable et leurs lipides ne contiennent pas ou peu de cholestérol.

Enfin, les lipides produits par les microalgues ont un profil d'acides gras plus simple que celui des huiles de poissons, ce qui limite les étapes de séparation des acides gras d'intérêt.

A l'heure actuelle, la classification des algues se fonde encore largement sur des critères morphologiques et sur la nature des pigments photosynthétiques que contiennent leurs cellules. De ce fait, elle est peu indicative du caractère autotrophe, hétérotrophe ou mixotrophe des espèces d'algues, alors que ces dernières recouvrent une très grande diversité d'espèces et de formes [Dubinsky et al. (2010); Hydrobiologia, 639:153-171].

La classification taxonomique des algues eucaryotes contient 14 phylums. Parmi les espèces des différentes classes composant ces phylums, qui produisent des acides gras, il existe des variations importantes en ce qui concerne la teneur des microalgues en acides gras polyinsaturés. Par ailleurs, les proportions relatives de lipides, notamment d'EPA, de DHA et d'ARA dans les profils lipidiques, varient selon l'espèce et les conditions de culture.

D'autre part, les caroténoïdes sont également des molécules d'intérêt. Ils sont généralement utilisés en tant que pigments, mais ils ont aussi un rôle important pour la santé de l'homme en tant qu'agents antioxydants. Enfin, ils présentent la capacité de stimuler le système immunitaire. La fucoxanthine est un exemple d'un caroténoïde, et est notamment contenue dans le wakame, algue utilisée dans la cuisine japonaise.

Pour mettre en oeuvre la production des acides gras et/ou de carotenoïde(s) par des microalgues à l'échelle industrielle, plusieurs facteurs doivent être pris en compte. Par exemple, les cultures peuvent être réalisées en condition autotrophe, mixotrophe ou hétérotrophe selon la souche, la température, les conditions de lumière et la taille des fermenteurs. Par exemple, les cultures peuvent également être réalisées dans des conteneurs d'un litre, dans un laboratoire, dans des photobioréacteurs, et dans des conteneurs de 100 000 litres ou bien dans des bassins ouverts (plusieurs hectares). Toutefois, les dépenses énergétiques et autres ressources telles que la main d'oeuvre et la facilité de poursuivre la culture doivent être prises en compte en développant des conditions de culture idéales.

En tout état de cause, il est souhaitable que les microalgues soient cultivées dans des conditions optimales pour augmenter le rendement de(s) l'acide(s) gras et du ou des caroténoïde(s) à produire. Ainsi, il est préférable d'avoir un rendement le plus élevé possible (par exemple, une biomasse au-delà de 30 g/l de matière sèche, et plus de 20 % d'acides gras en poids par rapport aux lipides totaux). Pour des caroténoïdes, un rendement au-delà de 0,2 % en matière sèche de microalgue est souhaitable.

Les microalgues du genre *Nitzschia* sont des diatomées marines que l'on trouve généralement dans les eaux froides telles que celles de l'Arctique ou de l'Antarctique. Ces microalgues sont connues principalement pour la production d'acide eicosapentaènoïque (EPA) en mode hétérotrophe.

On note toutefois que le mode mixotrophe a été étudié sur l'espèce *Nitzschia laevis* afin d'évaluer son impact sur la synthèse d'EPA (Biotechnology Letters, 2000, 22(9):727-733). Ainsi, c'est au terme de nombreuses expérimentations dans des conditions de lumière inhabituelles et par l'adjonction de différents substrats que le demandeur est parvenu à isoler des souches de microalgue de l'espèce *Nitzschia brevirostris,* cultivables en mode mixotrophe permettant, dans les conditions de la présente invention, une production à haut rendement d'acides gras polyinsaturés, en particulier de DHA et/ou d'EPA, et/ou de fucoxanthine.

Une souche (FCC 810) représentative des nouvelles souches de *Nitzschia brevirostris* ainsi isolées et sélectionnées, a été déposée auprès de la CCAP (Culture Collection of Algae and Protozoa, Scottish Association for Marine Science, Dunstaffnage Marine Laboratory, Oban, Argyll PA371QA, Ecosse, Royaume-Uni) selon les dispositions du Traité de Budapest, sous le numéro d'accession CCAP 1052/21.

Le procédé de culture et de sélection a consisté plus particulièrement à cultiver les microalgues en conditions de mixotrophie, en présence d'un éclairement variable et/ou discontinu, notamment sous forme de flashs, avec une gamme de variations d'intensité lumineuse et une fréquence spécifiques.

L'alternance rapprochée de phases éclairées et de phases obscures ou de moindre intensité lumineuse, perçue généralement comme stressante par les microalgues, a permis, de façon surprenante, d'obtenir des souches de *Nitzschia brevirostris* ayant une production élevée de biomasse, de lipides et plus particulièrement d'acides gras polyinsaturés, et/ou du ou des caroténoïde(s), en particulier de la fucoxanthine. Cette mise en oeuvre des souches selon l'invention ouvre la perspective d'une production industrielle d'acides gras polyinsaturés, en particulier de DHA et/ou d'EPA, et/ou de la fucoxanthine, dans des fermenteurs bénéficiant d'un apport lumineux réduit, et devrait donc permettre de réaliser des économies d'énergie par rapport aux modes de culture autotrophes.

Les différents aspects et avantages de l'invention sont détaillés ci-après.

### Description détaillée

La présente invention a donc pour objet un procédé de culture des microalgues du genre *Nitzschia,* notamment de l'espèce *Nitzschia brevirostris,* en mode mixotrophe dans des conditions d'éclairement discontinu et/ou variable au cours du temps, l'éclairement présentant des variations d'intensité entre les phases éclairées et les phases obscures dont l'amplitude est comprise entre 30 µmol. m⁻². s⁻¹ et 1000 µmol. m⁻². s⁻¹, de préférence, entre 30 et 400 µmol. m⁻². s⁻¹. Ces variations ayant lieu entre 2 et 3600 fois par heure, de préférence, entre 2 et 200 fois par heure. Le procédé selon l'invention permet un enrichissement des microalgues du genre *Nitzschia* en acides gras polyinsaturés, plus particulièrement en DHA et/ou en EPA, et/ou en caroténoïde(s), plus particulièrement en fucoxanthine.

Ces conditions de culture permettent d'apporter une quantité définie de lumière. Cet apport lumineux peut comporter des phases d'éclairement discontinu et/ou variable, avec des variations d'intensité pouvant avoir des amplitudes identiques ou différentes. L'éclairement peut être notamment sous forme de flashs.

Ce procédé a pour avantage d'augmenter le rendement en biomasse obtenu de la culture. Il a aussi pour avantage d'enrichir les microalgues ainsi cultivées en acides gras polyinsaturés, plus particulièrement en acide docosahexaénoïque (DHA) et/ou en acide éicosapentaénoïque (EPA), et/ou en caroténoïde(s), plus particulièrement en fucoxanthine. Ce procédé peut être également utilisé, pour sélectionner des souches du genre *Nitzschia,* en particulier de l'espèce *Nitzschia brevirostris,* à caractère mixotrophe, et ayant un haut rendement en acides gras polyinsaturés, notamment en DHA et/ou en EPA, et ou en caroténoïde(s), plus particulièrement en fucoxanthine.

La culture en mode mixotrophe de cette microalgue s'effectue préférentiellement en présence de 5 mM à 1 M, de préférence de 50 mM à 800 mM, plus préférentiellement de 70 mM à 600 mM, et encore plus préférentiellement de 100 mM à 500 mM d'un substrat carboné organique. L'apport du substrat est assuré continuellement pendant la culture, afin de permettre aux cellules d'accumuler une concentration importante de lipides. Du substrat additionnel est ajouté au milieu de culture pendant le procédé de culture pour maintenir une concentration constante. Ce substrat carboné organique comprend préférentiellement, sous forme pure ou en mélange : du glucose, des dérivés de cellulose, de lactate, du lactose, du saccharose, de l'acétate et/ou du glycérol.

Le substrat carboné organique contenu dans le milieu de culture peut consister en des molécules complexes ou en un mélange de substrats. Les produits issus de la biotransformation de l'amidon, par exemple à partir de maïs, de blé ou de pomme de terre, notamment les hydrolysats de l'amidon, qui sont constitués de molécules de petite taille, constituent, par exemple, des substrats carbonés organiques adaptés à la culture en mixotrophie des microalgues selon l'invention.

Ce procédé est plus particulièrement destiné à la mise en oeuvre de nouvelles souches de microalgues du genre *Nitzschia* (Division : Bacillariophyta, Ordre : Bacillariales, Famille : Bacillariaceae) [ITIS Catalogue of Life, 2010] sélectionnées pour leur caractère mixotrophe, notamment pour leur capacité à être cultivées avec un apport lumineux supérieur à 10 µE, dans un milieu minéral, par exemple, milieu f plus silice enrichie en azote [Guillard, R.R.L. (1975). Culture of phytoplankton for feeding marine invertebrates, dans Culture of Marine Invertebrate Animals, pp 26-60. Smith W.L. and Chanley M.H (Eds.) Plenum Press, New York], dans lequel est ajouté un substrat carboné organique. De préférence, le substrat carboné organique comprend du glucose, ou du lactate, dans une concentration équivalente ou supérieure à 5 mM.

Ces nouvelles souches de *Nitzschia,* plus particulièrement de *Nitzschia brevirostris* peuvent être isolées et sélectionnées selon le procédé de sélection et de culture selon l'invention décrit plus loin.

Une souche représentative des souches de *Nitzschia* selon l'invention est la souche FCC 810 isolée par le demandeur et déposée à la CCAP, sous le numéro CCAP 1052/21. De telles souches sont capables de produire des quantités significatives de biomasse ainsi que des lipides et plus particulièrement du DHA et/ou de l'EPA quand elles sont cultivées en mode mixotrophe avec un apport en lumière variable ou discontinu, selon l'invention. Elles sont également capables de produire des quantités significatives de caroténoïde(s), plus particulièrement de la fucoxanthine.

Selon les analyses taxonomiques réalisées, la souche CCAP 1052/21 appartient à l'espèce *Nitzschia brevirostris.*

Les souches de *Nitzschia brevirostris* isolées permettent de produire, en condition de mixotrophie, des quantités significatives de biomasse ainsi que de lipides riches en DHA et/ou EPA, ledit DHA ou EPA pouvant représenter plus de 20 %, plus de 25 % ou plus de 30 % des lipides totaux contenus dans les microalgues. Egalement les souches de *Nitzschia brevirostris* isolées permettent de produire, en condition de mixotrophie, des quantités significatives de caroténoïde(s), plus particulièrement de la fucoxanthine, pouvant représenter plus de 0,2 % en matière sèche, plus préférentiellement plus de 0,25 % en matière sèche sur la matière sèche totale de microalgue (ce qui inclut la teneur en caroténoïdes contenus dans ladite microalgue).

Dans la présente invention, la biomasse obtenue avec la souche FCC 810, isolée par le demandeur, d'une culture en conditions mixotrophes en présence d'un éclairement variable et/ou discontinu, notamment sous forme de flashs, est de 10 à 60 %, plus généralement de 20 à 50 %, supérieure à celle d'une culture avec la même souche effectuée en mode hétérotrophe. Par mode hétérotrophe, on entend des conditions de culture avec un milieu de culture identique, mais sans apport de lumière.

L'invention a ainsi pour objet un procédé de culture de microalgues du genre *Nitzschia,* notamment de l'espèce *Nitzschia brevirostris* en mode mixotrophe, en présence d'un éclairement variable ou discontinu au cours du temps, par exemple sous forme de flashs, notamment en vue de produire des acides gras polyinsaturés, tels que le DHA et/ou EPA, et/ou un ou des caroténoïde(s), tel que de la fucoxanthine.

Il est apparu qu'un éclairement variable et/ou discontinu des cultures, en particulier dans la mise en oeuvre d'une culture en mode mixotrophe, avait un impact favorable sur le développement des algues et permettait d'accroître la productivité de celles-ci, notamment en ce qui concerne leur production de lipides et/ou des caroténoïdes. Sans être lié par la théorie, l'inventeur estime qu'un apport discontinu et/ou variable de lumière aux microalgues a pour effet de provoquer un « stress » favorable à la croissance et à la synthèse des lipides et/ou des caroténoïdes. Ce phénomène pourrait s'expliquer, en partie, par le fait que dans la nature, les microalgues ont tendance à accumuler des réserves lipidiques et/ou en caroténoïdes pour résister aux contraintes de leur environnement.

Par éclairement discontinu, il faut entendre un éclairement ponctué par des périodes d'obscurité. Les périodes d'obscurité peuvent occuper plus d'un quart du temps, de préférence la moitié du temps ou plus, durant lequel les algues sont cultivées.

Selon un aspect préféré de l'invention, l'éclairement est discontinu et plus préférentiellement sous forme de flashs. Un flash, au sens de l'invention, est un éclairement lumineux de courte durée, c'est-à-dire de moins de 30 minutes. La durée du flash peut être de moins de 15 minutes, de préférence de moins de 5 minutes ou plus préférentiellement encore de moins de 1 minute. Selon certains modes de réalisation de l'invention, la durée du flash peut être de moins d'une seconde. Par exemple, le durée du flash peut être de 1/10 d'une seconde, ou de 2/10 d'une seconde, ou de 3/10 d'une seconde, ou de 4/10 d'une seconde ou de 5/10 d'une seconde, ou de 6/10 d'une seconde, ou de 7/10 d'une seconde, ou de 8/10 d'une seconde, ou de 9/10 d'une seconde. L'éclairement lumineux, ou le flash, est généralement d'une durée supérieure à 15 secondes. La durée du flash est généralement comprise entre 5 secondes et 10 minutes, de préférence entre 10 secondes et 2 minutes, plus préférentiellement entre 20 secondes et 1 minute. Selon l'invention, le nombre de flashs est compris entre environ 2 et 3600 par heure. Il peut être, par exemple, compris entre 100 et 3600 flashs par heure. Il peut être également compris entre 120 et 3000, ou entre 400 et 2500, ou entre 600 et 2000, ou entre 800 et 1500 flashs par heure. Il peut être également compris entre 2 et 200, préférentiellement entre 10 et 150, plus préférentiellement entre 15 et 100, et plus préférentiellement encore entre 20 et 50 par heure. Les flashs peuvent être émis à intervalle régulier ou non dans le temps. En cas d'émission à intervalle régulier, le nombre de flashs par heure correspond alors à une fréquence (F) ayant une période temporelle (T), étant considéré que F = 1/T. Cette période temporelle peut être comprise entre 1 seconde et 30 minutes, ou entre 1 seconde et 36 secondes, ou encore entre 1,2 seconde et 30 secondes, ou entre 1,44 seconde et 9 secondes, ou entre 1,8 seconde et 6 secondes, ou entre 2,4 secondes et 4,5 secondes. Cette fréquence peut être également comprise entre 18 secondes et 30 minutes, préférentiellement entre 24 secondes et 6 minutes, plus préférentiellement entre 36 secondes et 4 minutes, et plus préférentiellement encore entre 72 secondes et 3 minutes. Le nombre de flashs par heure est choisi en fonction de l'intensité et la durée des flashs (voir ci-dessous). Selon l'invention, l'intensité de la lumière apportée sous forme de flashs est entre 30 et 1000 µmol. m⁻². s⁻¹, de préférence entre 30 et 500 µmol. m⁻². s⁻¹, ou 50 et 400 µmol. m⁻². s⁻¹, et plus préférentiellement entre 150 et 300 µmol. m⁻². s⁻¹. Par définition, 1 µmol. m⁻². s⁻¹ correspond à 1µE m⁻². s⁻¹ (Einstein), unité souvent utilisée dans la littérature.

Selon un mode particulier de l'invention, l'intensité de la lumière est comprise entre 50 et 200 µmol. m⁻². s⁻¹, la période temporelle de la fréquence des flashs est comprise entre 10 secondes et 60 minutes pour une durée de flash comprise entre 1 seconde et 1 minute.

Selon l'invention, quelles que soient les conditions d'éclairement, l'intensité lumineuse apportée aux algues en culture, exprimée en micromoles de photons par seconde par mètre carré (µmol. m⁻². s⁻¹), varie au moins une fois dans une même heure. L'amplitude de cette variation d'intensité de lumière est généralement entre 30 et 1000, ou entre 50 et 800, ou entre 100 et 600 µmol. m⁻². s⁻¹. L'intensité de la lumière peut également varier, entre 30 et 400 µmol. m⁻². s⁻¹. De préférence, l'amplitude de la variation d'intensité de la lumière est entre 70 et 300 µmol. m⁻². s⁻¹ et plus préférentiellement entre 100 et 200 µmol. m⁻². s⁻¹.
Alternativement, en conditions d'éclairement discontinu, ladite intensité lumineuse peut atteindre successivement, plusieurs fois dans l'heure, par exemple, les valeurs 0 et 50 µmol. m⁻². s⁻¹, les valeurs 0 et 100 µmol. m⁻². s⁻¹ ou préférentiellement encore les valeurs 0 et 200 µmol. m⁻². s⁻¹. Elle peut également atteindre successivement, plusieurs fois dans l'heure, par exemple, les valeurs 0 et 300 µmol. m⁻². s⁻¹, les valeurs 0 et 600 µmol. m⁻². s⁻¹, les valeurs 0 et 800 µmol. m⁻². s⁻¹ ou encore les valeurs 0 et 1000 µmol. m⁻². s⁻¹.

Selon un mode de l'invention et quelles que soient les conditions d'éclairement, l'intensité de la lumière apportée à la culture varie en fonction de la densité cellulaire. Plus la culture devient dense, plus la lumière peut être intense. La densité cellulaire est le nombre de cellules par ml et est mesurée selon les techniques connues de l'homme de l'art.
Quand la culture atteint une densité entre 10⁶ et 10⁷ cellules par ml, l'intensité lumineuse peut être augmentée jusqu'à entre 30 et 500 µmol. m⁻². s⁻¹, par exemple, de préférence, entre 50 et 400 µmol. m⁻². s⁻¹. Quand la culture, au stade final, atteint une densité entre 10⁷ et 10⁸ cellules par ml, l'intensité lumineuse peut être augmentée jusqu'à entre 100 et 1000 µmol. m-². s⁻¹ par exemple, de préférence, entre 200 et 500 µmol. m⁻². s⁻¹.

Selon un mode de l'invention, la quantité de lumière apportée à la culture dans l'heure reste entre certaines valeurs. Elle est comprise entre environ 2000 et 600 000, de préférence entre 2000 et 300 000 µmol. m⁻². Elle peut être comprise entre environ 4000 et 200 000 µmol. m⁻², par heure.

Selon un autre mode de l'invention, la culture est illuminée avec 120 flashs par heure, chaque flash ayant une durée de 10 secondes et une intensité de 200 µmol. m⁻². s⁻¹, ce qui donne un apport total de lumière par heure de 240 000 µmol. m⁻².

Comme décrit pour l'intensité lumineuse ci-dessus, et selon un mode de l'invention, la quantité de lumière apportée à la culture par heure peut varier en fonction de la densité cellulaire. Au stade initial de la culture quand la densité cellulaire est 10⁵ et 5 x 10⁵ cellules par ml, l'apport total de lumière dans l'heure est généralement compris entre environ 1500 et 8000, de préférence 1500 et 6000 µmol. m⁻², de préférence encore entre 2000 et 5000 µmol. m⁻². Quand la culture atteint une densité entre 10⁶ et 10⁷ cellules par ml, l'apport total de lumière dans l'heure peut être augmenté jusqu'à entre 6000 et 67 000 µmol. m⁻², de préférence, entre 6000 et 50 000 et de préférence encore entre 12 000 et 45 000 µmol. m⁻², par exemple. Au stade final de la culture, à une densité cellulaire entre 10⁷ et 10⁸ cellules par ml, l'apport total de lumière dans l'heure peut être augmenté jusqu'à entre 45 000 et 300 000, par exemple, de préférence, entre 45 000 et 200 000 µmol. m⁻², et par exemple, de préférence encore, entre 50 000 et 150 000 µmol. m⁻². Au stade initial de la culture (à une densité cellulaire entre 10⁵ et 5 x 10⁵ cellules par ml), la culture est illuminée avec 30 flashs par heure, chaque flash ayant une durée de 30 secondes et une intensité entre 5 et 10 µmol. m⁻². s⁻¹, ce qui donne un apport total de lumière par heure de 2250 µmol. m⁻² à 4500 µmol. m⁻². Puis, au stade intermédiaire (à une densité cellulaire entre 10⁶ et 10⁷ cellules par ml), la culture est illuminée avec 30 flashs par heure, chaque flash ayant une durée de 30 secondes et une intensité entre 15 et 50 µmol. m⁻². s⁻¹, ce qui donne un apport total de lumière par heure de 13 500 à 45 000 µmol. m⁻². Puis, au stade final de la culture (à une densité cellulaire entre entre 10⁷ et 10⁸ cellules par ml), la culture est illuminée avec 30 flashs par heure, chaque flash ayant une durée de 30 secondes et une intensité entre 50 et 150 µmol. m⁻². s⁻¹, ce qui donne un apport total de lumière par heure de 45 000 à 135 000 µmol. m⁻².

Selon un mode de l'invention, par exemple, quand la durée des flashs est par exemple de moins d'une minute, ou moins d'une seconde, au stade initial de la culture (à une densité cellulaire entre 10⁵ et 5 x10⁵ cellules par ml), la culture est illuminée avec 30 flashs par heure, chaque flash ayant une durée de 10 secondes et une intensité entre 50 et 100 µmol. m⁻². s⁻¹, ce qui donne un apport total de lumière par heure de 15 000 µmol. m⁻² à 30 000 µmol. m⁻². Puis, au stade intermédiaire (à une densité cellulaire entre 10⁶ et 10⁷ cellules par ml), la culture est illuminée avec 50 flashs par heure, chaque flash ayant une durée de 10 secondes et une intensité entre 200 et 300 µmol. m⁻². s⁻¹, ce qui donne un apport total de lumière par heure de 100 000 à 150 000 µmol. m⁻². Puis, au stade final de la culture (à une densité cellulaire entre entre 10⁷ et 10⁸ cellules par ml), la culture est illuminée avec 120 flashs par heure, chaque flash ayant une durée de 10 secondes et une intensité entre 350 et 450 µmol. m⁻². s⁻¹, ce qui donne un apport total de lumière par heure de 420 000 à 540 000 µmol. m⁻².

L'apport de lumière dans les cultures peut être obtenu par des lampes réparties autour de la paroi externe des fermenteurs. Une horloge déclenche ces lampes pour des temps d'éclairement définis. Les fermenteurs se situent préférentiellement dans une enceinte à l'abri de la lumière du jour, dont on peut contrôler la température ambiante.

Ainsi qu'a pu le constater le déposant, le fait que les souches ainsi sélectionnées présentent de bonnes aptitudes à croître en mode mixotrophe, en présence d'une lumière discontinue, prédispose lesdites souches à une production plus élevée d'acides gras polyinsaturés, notamment de DHA et/ou d'EPA, et/ou une production plus élevée de caroténoïde(s), plus particulièrement de fucoxanthine.

Le procédé de culture permet ainsi de sélectionner des souches du genre *Nitzschia,* en particulier de l'espèce *Nitzschia brevirostris* à caractère mixotrophe, similaires à celle isolée par le demandeur et déposée à la CCAP sous le numéro CCAP 1052/21, et ayant un haut rendement en acides gras polyinsaturés et/ou en caroténoïde(s). Ce procédé de culture est caractérisé en ce qu'il comprend des étapes suivantes :
a) la culture en mode mixotrophe d'une ou plusieurs souches du genre *Nitzschia* dans des conditions d'éclairement discontinu, l'éclairement présentant des variations d'intensité dont l'amplitude est comprise entre 30 µmol. m⁻². s⁻¹ et 1000, de préférence entre 30 et 400 µmol. m⁻². s⁻¹, ces variations ayant lieu entre 2 et 3600, de préférence 5-400 fois par heure,
b) une étape de maintien de ladite culture sur plusieurs générations, en présence d'un substrat carboné organique dans le milieu de culture, et éventuellement
c) une étape de récupération des microalgues ainsi cultivées.

Par étape de récupération, on entend plus particulièrement l'isolement de la ou des souches dont le nombre de cellules s'est accru le plus au cours desdites générations.

Avantageusement, la culture en mode mixotrophe s'effectue dans des conditions d'éclairement discontinu, l'éclairement présentant des variations d'intensité dont l'amplitude est comprise entre 30 µmol. m⁻². s⁻¹ et 400 µmol. m⁻². s⁻¹, ces variations ayant lieu entre 2 et 200 fois par heure.

Pour réaliser la sélection des souches, différentes souches du genre *Nitzschia,* en particulier de l'espèce *Nitzschia brevirostris,* peuvent être cultivées, en parallèle, sur des microplaques dans une même enceinte, avec un suivi précis des conditions et de l'évolution des différentes cultures. Il est ainsi aisé de connaître la réponse des différentes souches à l'éclairement discontinu et/ou variable et, le cas échéant, à l'adjonction d'un ou plusieurs substrats carbonés organiques dans le milieu de culture. Les souches qui répondent favorablement à l'éclairement discontinu et/ou variable et aux substrats carbonés organiques offrent généralement un meilleur rendement pour la production de caroténoïdes et de lipides sur le plan qualitatif (acides gras polyinsaturés plus abondants dans le profil lipidique et fucoxanthine plus abondant parmi les caroténoïdes) et quantitatif (les lipides contiennent une proportion plus élevée de DHA et/ou EPA).

Les microalgues peuvent être sélectionnées dans un fermenteur à partir d'une population hétérogène et dont on cherche à sélectionner les variants avantagés par le mode de sélection alliant lumière discontinue et/ou variable présentant une gamme d'intensité lumineuse et une fréquence spécifiques, avec des conditions de culture mixotrophes. Dans ce cas, la culture est pratiquée en maintenant les microalgues en cultures sur de nombreuses générations, puis un isolement des composantes devenues majoritaires dans le milieu de culture est effectué au terme de la culture.

Le procédé de culture selon l'invention permet également de produire des lipides.

Dans ce cas, le procédé selon l'invention comporte en outre les étapes suivantes :
d) une étape de récupération des lipides des microalgues et éventuellement
e) l'extraction du DHA et/ou de l'EPA des lipides récupérés.

Le procédé de culture selon l'invention permet également de produire des caroténoïdes.

Dans ce cas, le procédé selon l'invention comporte en outre les étapes suivantes :
d) une étape de récupération de la matière hydrophobe des microalgues et éventuellement
e) l'extraction du DHA et/ou de l'EPA et/ou de la fucoxanthine de la matière hydrophobe récupérée.

Le procédé de culture selon l'invention peut s'appliquer également à toute espèce du genre *Nitzschia,* capable de croître dans les conditions mixotrophes selon l'invention, et capable de produire le DHA et/ou de l'EPA, et/ou de la fucoxanthine.

Le procédé de culture selon l'invention permet d'optimiser la production de la biomasse obtenue de la culture. Il permet également d'enrichir les microalgues ainsi cultivées en acides gras polyinsaturés, plus particulièrement en DHA et/ou en EPA, et/ou d'enrichir les microalgues ainsi cultivées en caroténoïde(s), plus particulièrement en fucoxanthine.

L'invention a donc également pour but l'optimisation de la production de biomasse, ainsi que la production de lipides, notamment d'acides gras, via la culture de microalgues du genre *Nitzschia* à caractère mixotrophe, de préférence cultivées ou sélectionnées selon les procédés visés précédemment, puis la récupération des microalgues ainsi cultivées pour en extraire le contenu lipidique, en particulier le DHA et/ou l'EPA. L'invention a également pour but l'optimisation de la production en caroténoïde(s), plus particulièrement en fucoxanthine. Les souches de l'espèce *Nitzschia brevirostris* sont spécialement concernées.

Les méthodes d'extraction sélective des lipides dont le DHA sont connues de l'homme du métier et sont, par exemple, décrites par [Bligh, E.G. et Dyer, W.J. (1959); A rapid method of total lipid extraction and purification, Can. J. Biochem. Physiol., 37:911-917] et par [McCreary DK, Kossa WC, Ramachandran S, Kurtz RR. (1978), "A novel and rapid method for the preparation of methyl esters for gas chromatography: application to the determination of the fatty acids of edible fats and oils.", J Chromatogr Sci. 16(8):329-31.]

Les méthodes d'extraction et d'analyse des caroténoïdes, dont la fucoxanthine, sont connues de l'homme du métier et sont, par exemple, décrites par Wright et al. (1991) (S.W. Wright, S.W. Jeffrey, R.F.C. Mantoura, C.A. Llewellyn, T. Bjornland, D. Repeta, N. Welschmeyer : Improved HPLC method for the analysis of chlorophylls and carotenoids from marine phytoplankton. Marine ecology progress series: Vol. 77 : 183-196, 1991).

L'invention porte également sur la biomasse de microalgues du genre *Nitzschia,* susceptibles d'être obtenues selon le procédé de l'invention tel que précédemment décrit. Ces microalgues sont enrichies en acides gras polyinsaturés. Les lipides totaux de telles microalgues comprennent plus de 20 %, souvent plus de 25 % et parfois même plus de 30 % de DHA et/ou d'EPA par rapport au pourcentage total de lipides. Les microalgues comprennent un taux de fucoxanthine de plus de 0,2 % en matière sèche, préférentiellement plus de 0,25 % en matière sèche sur la matière sèche de microalgue.

### Exemple 1

Les cultures de *Nitzschia brevirostris* FCC 810 ont été réalisées dans des fermenteurs (bioréacteurs) de 2L utiles avec automates dédiés et supervision par station informatique. Le système est régulé en pH via l'ajout de base (solution d'hydroxyde de sodium à 1N) et/ou d'acide (solution d'acide sulfurique à 1N). La température de culture est fixée à 25°C. L'agitation est réalisée grâce à 2 mobiles d'agitation placés sur l'arbre selon la configuration suivante : hélice de Rushton et hélices tripales à pompage descendant. La vitesse d'agitation et le débit d'aération sont régulés au mini = 100 rpm et au maxi = 250 rpm et Qmini =0,5 wm / Qmaxi = 2 wm respectivement. Le bioréacteur est équipé d'un système luminaire externe entourant la cuve transparente.

Les réacteurs sont inoculés avec une pré-culture réalisée sur table d'agitation (140 rpm) en enceinte thermo statée (25°C) et éclairée entre 80 et 100 uE. Les pré-cultures et cultures en bioréacteurs sont réalisées dans le milieu F plus silice enrichie en azote. Le substrat carboné organique utilisé pour la culture en mixotrophie en bioréacteur est le glucose à des concentrations entre 100 mM et 150 mM.

### Suivi des cultures :

La concentration en biomasse totale est suivie par mesure de la masse sèche (filtration sur filtre GFB, Whatman, puis séchage à l'étuve à 100°C pendant 24h minimum avant pesée).

Concernant la quantification des lipides totaux, 7.10⁸ cellules/mL ont été extraites. Les méthodes d'extraction des lipides sont connues de l'homme du métier.

### Eclairement :

La culture est illuminée avec 180 flashs par heure, chaque flash ayant une durée de 5 secondes et une intensité de 300 µmol. m⁻². s⁻¹.

L'apport de lumière dans les cultures en bioréacteur a été obtenu par des LED (Diodes Electro Luminescentes) réparties autour de la paroi externe du fermenteur. Un pilotage informatique déclenche l'alimentation des LED pour des temps d'éclairement ou des flashs.

### Résultats :

| | Masse Sèche (g/L) | Lipides totaux (% de la MS) | % DHA | %EPA | Fucoxanthine (mg/g) |
|---|---|---|---|---|---|
| Mixotrophie Flash | 35,1 +/-0,7 | 23,6 +/-0,4 | 0,6 +/-0,5 | 26 +/- 1 | 2,2 +/- 0,1 |
| Hétérotrophie | 25,4 +/-0,2 | 24 +/- 0,3 | 0,8 +/-0,4 | 22 +/- 1 | 0,8 +/- 0,1 |

## Revendications

1. Procédé comprenant les étapes suivantes:
a) la culture en mode mixotrophe d'une ou plusieurs souches du genre *Nitzschia* dans des conditions d'éclairement discontinu et/ou variable au cours du temps, l'éclairement présentant des variations d'intensité entre les phases éclairées et les phases obscures dont l'amplitude est comprise entre 30 et 1000 µmol. m⁻².s⁻¹, ces variations ayant lieu entre 2 et 3600 fois par heure,
b) une étape de maintien de ladite culture sur plusieurs générations en présence d'un substrat carboné organique dans le milieu de culture, et
c) une étape de récupération des microalgues ainsi cultivées.

2. Procédé selon la revendication 1, **caractérisé en ce que** l'éclairement présente des variations d'intensité dont l'amplitude est comprise entre 30 µmol. m⁻².s⁻¹ et 400 µmol. m⁻².s⁻¹, ces variations ayant lieu entre 2 et 200 fois par heure.

3. Procédé selon la revendication 1 ou 2, **caractérisé en ce que** la microalgue est de l'espèce *Nitzschia brevirostris.*

4. Procédé selon la revendication 1, 2 ou 3 , **caractérisé en ce que** la culture s'effectue en présence d'un substrat carboné organique à une concentration de 5 mM à 1 M, de préférence de 50 mM à 800 mM, plus préférentiellement de 70 mM à 600 mM, et encore plus préférentiellement de 100 mM à 500 mM, le substrat carboné organique étant choisi parmi le lactate, le lactose, le saccharose, l'acétate, le glycérol, le glucose et les dérivés de cellulose et un mélange de ces molécules.

5. Procédé selon la revendication 4, **caractérisé en ce que** ledit substrat carboné organique présent dans le milieu de culture comprend au moins 5 mM de glucose.

6. Procédé selon la revendication 5, **caractérisé en ce que** l'amplitude des variations d'intensité est comprise entre 70 et 300 µmol. m⁻².s⁻¹, et plus préférentiellement entre100 et 200 µmol. m⁻².s⁻¹.

7. Procédé selon l'une quelconque des revendications 1 à 6, **caractérisé en ce que** l'apport de lumière s'effectue sous forme de flashs.

8. Procédé selon la revendication 7, **caractérisé en ce qu'**un flash a une durée comprise entre 1/10 seconde et 10 minutes, de préférence entre 5 secondes et 10 minutes, de préférence encore entre 10 secondes et 2 minutes, plus préférentiellement encore entre 20 secondes et 1 minute.

9. Procédé selon la revendication 7 ou la revendication 8, **caractérisé en ce que** le nombre de flashs est entre 5 et 3600, de préférence entre 10 et 150, plus préférentiellement entre 15 et 100, et plus préférentiellement encore entre 20 et 50 fois par heure.

10. Procédé selon l'une quelconque des revendications 1 à 8, **caractérisé en ce que** l'apport total de lumière par heure en micromoles des photons est entre 2000 à 600 000, de préférence entre 2000 à 200 000 µmol. m⁻².

11. Procédé selon l'une quelconque des revendications 1 à 10, **caractérisé en ce qu'**il comprend en outre les étapes suivantes:
d) une étape de récupération de la matière hydrophobe de la microalgue, suivie, le cas échéant,
e) de l'extraction du DHA et/ou de l'EPA et/ou de la fucoxanthine de la matière hydrophobe récupérée.

12. Procédé selon l'une quelconque des revendications 3 à 11, **caractérisé en ce que** ladite microalgue de l'espèce *Nitzschia brevirostris* correspond à la souche FCC 810, déposée auprès de la CCAP (Culture Collection of Algae and Protozoa), sous le numéro CCAP 1052/21.

13. Biomasse de microalgues du genre *Nitzschia,* susceptible d'être obtenue par le procédé de l'une des revendications 1 à 12, **caractérisée en ce qu'**elle comprend plus de 30 g/l de matière sèche et que ses lipides totaux comprennent plus de 20 %, plus de 25 % ou plus de 30 % d'EPA et/ou de DHA et **en ce qu'**elle comporte plus de 0,2 % en poids de fucoxanthine sur le poids total de matière sèche.

14. Biomasse selon la revendication 13, **caractérisée en ce que** les microalgues sont de l'espèce *Nitzschia brevirostris.*

## Patentansprüche

1. Verfahren, umfassend die folgenden Schritte:
a) die Kultur im mixotrophen Modus von einem oder mehreren Stämmen der Gattung *Nitzschia* unter diskontinuierlichen und/oder im Laufe der Zeit variablen Beleuchtungsbedingungen, wobei die Beleuchtung Intensitätsschwankungen zwischen den hellen Phasen und den dunklen Phasen aufweist, deren Amplitude zwischen 30 und 1000 µmol.m⁻².s⁻¹ inklusive beträgt, wobei diese Schwankungen zwischen 2 und 3600 Mal pro Stunde stattfinden,
b) einen Schritt des Haltens der Kultur über mehrere Generationen bei Anwesenheit eines organischen kohlenstoffhaltigen Substrats in dem Kulturmilieu, und
c) einen Schritt der Rückgewinnung der derart kultivierten Mikroalgen.

2. Verfahren nach Anspruch 1, **dadurch gekennzeichnet, dass** die Beleuchtung Intensitätsschwankungen aufweist, deren Amplitude zwischen 30 µmol.m⁻².s⁻¹ und 400 µmol.m⁻².s⁻¹ inklusive beträgt, wobei diese Schwankungen zwischen 2 und 200 Mal pro Stunde stattfinden.

3. Verfahren nach Anspruch 1 oder 2, **dadurch gekennzeichnet, dass** die Mikroalge die Spezies *Nitzschia brevirostris* ist.

4. Verfahren nach Anspruch 1, 2 oder 3, **dadurch gekennzeichnet, dass** die Kultur bei Anwesenheit eines organischen kohlenstoffhaltigen Substrats in einer Konzentration von 5 mM bis 1 M, vorzugsweise von 50 mM bis 800 mM, vorzugsweiser von 70 mM bis 600 mM und noch vorzugsweiser von 100 mM bis 500 mM erfolgt, wobei das organische kohlenstoffhaltige Substrat aus dem Lactat, der Lactose, der Saccharose, dem Acetat, dem Glycerol, der Glucose und den Zellulosederivaten und einem Gemisch dieser Moleküle ausgewählt ist.

5. Verfahren nach Anspruch 4, **dadurch gekennzeichnet, dass** das im Kulturmilieu vorhandene organische kohlenstoffhaltige Substrat mindestens 5 mM Glucose umfasst.

6. Verfahren nach Anspruch 5, **dadurch gekennzeichnet, dass** die Amplitude der Intensitätsschwankungen zwischen 70 und 300 µmol.m⁻².s⁻¹ und vorzugsweiser zwischen 100 und 200 µmol. m⁻².s⁻¹ inklusive beträgt.

7. Verfahren nach einem der Ansprüche 1 bis 6, **dadurch gekennzeichnet, dass** die Zufuhr von Licht in Form von Blitzen erfolgt.

8. Verfahren nach Anspruch 7, **dadurch gekennzeichnet, dass** ein Blitz eine Dauer zwischen 1/10 Sekunde und 10 Minuten, vorzugsweise zwischen 5 Sekunden und 10 Minuten, vorzugsweise auch zwischen 10 Sekunden und 2 Minuten, noch vorzugsweiser zwischen 20 Sekunden und 1 Minute hat.

9. Verfahren nach Anspruch 7 oder Anspruch 8, **dadurch gekennzeichnet, dass** die Anzahl von Blitzen zwischen 5 und 3600, vorzugsweise zwischen 10 und 150, vorzugsweiser zwischen 15 und 100 und noch vorzugsweiser zwischen 20 und 50 Mal pro Stunde beträgt.

10. Verfahren nach einem der Ansprüche 1 bis 8, **dadurch gekennzeichnet, dass** die Gesamtzufuhr von Licht pro Stunde in Photonen-Mikromol zwischen 2000 bis 600000, vorzugsweise zwischen 2000 bis 200000 µmol.m⁻², beträgt.

11. Verfahren nach einem der Ansprüche 1 bis 10, **dadurch gekennzeichnet, dass** es ferner die folgenden Schritte umfasst:
d) einen Schritt der Rückgewinnung des hydrophoben Materials aus der Mikroalge, gefolgt, gegebenenfalls
e) von der Extraktion von DHA und/oder von EPA und/oder von Fucoxanthin aus dem rückgewonnenen hydrophoben Material.

12. Verfahren nach einem der Ansprüche 3 bis 11, **dadurch gekennzeichnet, dass** die Mikroalge der Spezies *Nitzschia brevirostris* dem Stamm FCC 810 entspricht, hinterlegt bei der CCAP (Culture Collection of Algae and Protozoa) unter der Nummer CCAP 1052/21.

13. Biomasse von Mikroalgen der Gattung *Nitzschia,* die anhand des Verfahrens nach einem der Ansprüche 1 bis 12 gewinnbar ist, **dadurch gekennzeichnet, dass** sie über 30 g/l Trockenmasse umfasst und dass ihre Lipide insgesamt über 20 %, über 25 % oder über 30 % EPA und/oder DHA umfassen und dass sie über 0,2 Gew.-% Fucoxanthin in Bezug zum Gesamtgewicht der Trockenmasse aufweist.

14. Biomasse nach Anspruch 13, **dadurch gekennzeichnet, dass** die Mikroalgen der Spezies *Nitzschia brevirostris* entsprechen.

## Claims

1. A method comprising the following steps:
a) culture, in mixotrophic mode, of one or more strains of the genus *Nitzschia* under conditions of illumination that is discontinuous and/or variable over time, the illumination having variations in intensity between the illuminated phases and the phases of darkness, the amplitude of which is comprised between 30 and 1,000 µmol ·m⁻²·s⁻¹, these variations taking place between 2 and 3,600 times per hour.
b) a step of maintaining said culture over several generations in the presence of an organic carbon-containing substrate in the culture medium, and
c) a step of recovery of the thus cultured microalgae.

2. The method according to claim 1, **characterized in that** the illumination has variations in intensity, the amplitude of which is comprised between 30 µmol ·m⁻²·s⁻¹ and 400 µmol ·m⁻²·s⁻¹, these variations taking place between 2 and 200 times per hour.

3. The method according to claim 1 or 2, **characterized in that** the microalga is from the species *Nitzschia brevirostris.*

4. The method according to claim 1, 2 or 3, **characterized in that** the culture is carried out in the presence of an organic carbon-containing substrate at a concentration from 5 mM to 1 M, preferably from 50 mM to 800 mM, more preferentially from 70 mM to 600 mM, and even more preferentially from 100 mM to 500 mM, the organic carbon-containing substrate being selected from lactate, lactose, sucrose, acetate, glycerol, glucose and cellulose derivatives and a mixture of these molecules.

5. The method according to claim 4, **characterized in that** said organic carbon-containing substrate present in the culture medium comprises at least 5 mM of glucose.

6. The method according to claim 5, **characterized in that** the amplitude of the variations in intensity is comprised between 70 and 300 µmol ·m⁻² ·s⁻¹ and more preferentially between 100 and 200 µmol ·m⁻²·s⁻¹.

7. The method according to any one of claims 1 to 6, **characterized in that** the light supply is in the form of flashes.

8. The method according to claim 7, **characterized in that** a flash has a duration comprised between 1/10 second and 10 minutes, preferably between 5 seconds and 10 minutes, more preferably between 10 seconds and 2 minutes, yet more preferentially between 20 seconds and 1 minute.

9. The method according to claim 7 or claim 8, **characterized in that** the number of flashes is between 5 and 3,600, preferably between 10 and 150, more preferentially between 15 and 100, and even more preferentially between 20 and 50 times per hour.

10. The method according to any one of claims 1 to 8, **characterized in that** the total light supply per hour in micromoles of photons is between 2,000 to 600,000, preferably between 2,000 to 200,000 µmol ·m⁻².

11. The method according to any one of claims 1 to 10, **characterized in that** it further comprises the following steps:
d) a step of recovery of the hydrophobic matter from the microalga, followed, if appropriate,
e) the extraction of the DHA and/or of the EPA and/or the fucoxanthin from the recovered hydrophobic matter.

12. The method according to any one of claims 3 to 11, **characterized in that** said microalga of the species *Nitzschia brevirostris* corresponds to the strain FCC 810, deposited with the CCAP (Culture Collection of Algae and Protozoa), under the number CCAP 1052/21.

13. A biomass of microalga of the genus *Nitzschia* which can be obtained by the method of one of claims 1 to 12, **characterized in that** it comprises 30 g/l of dry matter and that its total lipids comprise more than 20%, more than 25% or more than 30% of EPA and/or of DHA and **in that** it comprises more than 0.2% by weight of fucoxanthin of the total weight of dry matter.

14. The biomass according to claim 13, **characterized in that** the microalgae are from the species *Nitzschia brevirostris.*
